# EUROPEAN PATENT APPLICATION

(11) **EP 3 616 724 A1**
(43) Date of publication of application: **04.03.2020**
(21) Application number: 18191027.4
(22) Date of filing: 27.08.2018
(51) Int. Cl.: A61K 47/54, A61K 47/59, A61K 47/69, A61P 1/16

(54) **PKC INHIBITORS FOR THE TREATMENT OF SEPTIC CHOLESTASIS WITH CTM TARGETING**

(71) Applicant: SmartDyeLivery GmbH, 07743 Jena (DE)
(72) Inventor: ENZENSPERGER, Christoph, 07745 Jena (DE); LEHMANN,Marc, 07745 Jena (DE)
(74) Representative: Ettmayr, Andreas

(57) **Abstract**

**Summary**

The invention relates to inhibitors of the PKC signaling pathway for use in the treatment of septic cholestasis, wherein the inhibitors are targeted into the liver by a selective nanostructured delivery system, wherein the selective nanostructured delivery system comprises at least one carbohydrate targeting moiety and at least one polymer and/or at least one lipid and/or at least one virus-like particle.

## Description

### Cholestasis

Cholestasis refers to an impaired bile formation and flow, and subsequent bilirubin and bile acid retention. Two forms of cholestasis are known: *extrahepatic cholestasis*, which is an obstructive type of cholestasis caused by a mechanical blockage in the duct system and displacement of the biliary tract which can occur, for example, from a gallstone, tumors, such as pancreatic carcinoma, bile duct cysts, bile duct stenosis or parasites, and intrahepatic cholestasis (non-obstructive cholestasis), where the reason for the congestion of bile is inside the liver. *Intrahepatic cholestasis* can occur because of genetic defects or can be acquired as a side effect of many medications, for example, non-steroidal anti-inflammatory drugs (NSAIDs), antihypertensive-, antidiabetic-, anticonvulsant-, lipid-lowering agents, anabolic steroids, psychotropic drugs, and various antibiotics. Moreover, cholestasis can also occur as a result of viral or alcoholic hepatitis, hepatocellular carcinoma, granulomatous liver disease, or liver cirrhosis. However, the second most prevalent cause is extrahepatic infection (sepsis). It can also occur during parenteral nutrition, pregnancy, after liver transplantation. As a consequence of the reduced bilirubin excretion, patients with cholestasis show symptoms of jaundice. Depending on the cause, cholestasis may be associated with itchiness (pruritus), gastrointestinal complaints, such as pale stool, dark urine, nausea, vomiting, and pain. Cholestatic liver disease is diagnosed by a predominant elevation of serum alkaline phosphatase and bilirubin, although serum bilirubin may be normal until a late stage of the disease. *Manifest intrahepatic cholestasis* is a rare, but typical symptom of sepsis. In Germany, for example, about 30.000 people develop a sepsis-associated organ-failure and 3-6% of them develop a condition called septic cholestasis or sepsis-induced cholestasis, which is characterized by additional symptoms of jaundice. The mortality is 92% during the first 12 months after diagnosis, and is much higher than for any other sepsis-associated organ failure. (Jaeger et al. Jaundice Increases the Rate if Complications and One-Year Mortality in Patients With Hypoxic Hepatitis, Hepatology 2012, 56(6), 2297)

### Sepsis

Sepsis should be defined as life threatening organ dysfunction caused by a dysregulated host response to infections with pathogens and is a major public health concern, accounting for more than $20 billion of total US hospital costs in 2011. It can be assumed that sepsis is a leading cause of mortality and critical illness worldwide. Furthermore, patients who survive sepsis often have long term physical, psychological and cognitive disabilities with significant health care and social implications. Pathogens responsible for the development of sepsis can have various origins and can arise from bacterial, viral, fungal or protozoal infections. Sepsis is not only defined by a systemic inflammation, more important is a dysregulated response of the patient to this infection, which leads to organ dysfunction and underlines the overall severity of this condition. Sepsis also involves pro- and anti-inflammatory responses, modifications in cardiovascular, neural, autonomic, hormonal, bioenergetics, metabolic processes and coagulation. Finally, the organ dysfunction or failure is responsible for the high sepsis associated mortality rate. Therefore, the so called "Sequential Organ Failure Assessment" (SOFA) scoring rate was developed to determine the severity of sepsis. They can be defined by clinical parameters as Pa_{O2}/Fi_{O2}, number of platelets, Bilirubin, Creatinine, urine output and mental status of the patient. It could be shown that an early antibiotic eradication of the underlying infection is most important for the survival of the patients. Additional therapeutic considerations include: avoiding parenteral nutrition, avoiding of hepatotoxic medications, monitoring of the glucose levels and adequate supply if necessary, extracorporal liver support (i.e. albumin dialysis). Immunocompromised patients e.g. after organ transplantation, cancer therapy or therapy of autoimmune diseases have increased risk of infection by common pathogens, as well as opportunistic infections by less virulent microorganisms of little concern to patients with a non-compromised immune system. So it is obvious that this highly developing risk of infection predisposes such individuals to increased risk of sepsis and septic shock.

In sepsis, the dysregulated host response to the systemic infection often leads to hepatocellular dysfunction of membranous transport processes with consecutive disorders of biliary excretion (Zollner G., Trauner M.; Mechanism of cholestasis, Clin Liver Dis 2008; 12: 1-26). As a consequence, in septic patients, jaundice can be observed as a consequence of an intrahepatic (non-obstructive) cholestasis. A challenge is to timely discriminate between sepsis-associated and other sepsis-unrelated causes of cholestasis, (*vide supra*). Usually, prior to the development of septic cholestasis, the manifestations of sepsis dominate the clinical picture. It is likely that uncontrolled infection leads to decreased function and expression of important hepatocyte transport proteins, leading to reduction of bilirubin excretion and jaundice (Zollner G., Trauner M., *I.c.*)*.* Septic cholestasis is associated with liver failure and has a mortality rate of over 92%.

### Sepsis-induced cholestasis

Sepsis-induced cholestasis is a special kind of excretory dysfunctions of the liver. Excretory dysfunctions of the liver can have various causes: From carcinoma, cysts in the gall duct, inflammation of the liver (e.g. hepatitis), fibrosis or cirrhosis, fatty liver (alcoholic or non-alcoholic), side effects of certain drugs (e.g. anabolic drugs, antipsychotic drugs and certain antibiotics). In the case of septic cholestasis, an underlying systemic infection causes a disturbance of the whole immune system and triggers a secretory dysfunction if the liver. Therefore, sepsis-induced cholestasis represents the complication of a systemic infection. At present, the only effective treatment of septic cholestasis is a treatment of the underlying sepsis by an antibiotic therapy of the infection which should be initiated as soon as possible. The window of opportunity for successful intervention is short and a delay in diagnosing infection and initializing antibiotic therapy significantly worsens the prognosis and survival chances of the patient (Fuchs M., Sanyal AJ.; Sepsis and cholestasis, Clin Liver Dis 2008; 12: 151-72). In order to promptly and efficiently eradicate a bacterial infection, usually a maximum tolerable dose of a combination of different broad spectrum antibiotics is used in therapy, because a diagnosis of the responsible pathogen (blood culture) is in most cases not possible within a timeframe that could be tolerated.

Antibiotic therapy of the systemic infection is also associated with known problems and certainly is no therapy of an organ failure, which results from the dysregulated response to the infection. Moreover, several antibiotics can disturb and block bile excretion and therefore induce cholestasis (*vide supra*). Most prominent examples include amoxicillin and erythromycin. The high dose broad spectrum antibiotic therapy causes an emerging incidence of pathogens with antibiotic resistance and hampers the success of future therapy. Additionally, it has to be considered that antibiotic therapy is pointless, or even counterproductive if the underlying infection is fungal, viral or protozoal, due to the potential unwanted (toxic) side effects of some antibiotics, such as allergy, interactions with food and other medicaments, and/or direct damages to major organs, mainly kidneys and liver, especially if the organ functions are compromised.

In addition to antibiotic therapy of sepsis, further treatment approaches of sepsis with kinase inhibitors were described. USH1168H, filed in 1991, describes a therapy for septic shock which reduces inflammation and improves tissue and organ perfusion comprising infusing a PKC inhibitor selected from the group consisting of lipid analogs. This means a systemic administration of PKC inhibitors. US 5,616,577, filed in 1996 (corresponding WO93/16703), describes the treatment and prevention of conditions wherein PKC inhibition is indicated. Those conditions are listed as cardiovascular and renal disorders, inflammation, central nervous system disorders, immunosuppression, and septic shock. US 2011/0130415 describes the treatment of a variety of inflammatory diseases including septic shock, by PKC inhibition.

While the effect of kinase inhibitors, especially PKC inhibition, on cholestasis is described (Anwer M.S.; Role of protein kinase C isoforms in bile formation and cholestasis, Hepatology 2014; 60(3): 1090-1097), treatment of sepsis by the use of kinase inhibitors is counterproductive and may cause life-threatening side effects for the reasons outlined in the paragraph "Kinase inhibitors and sepsis" (*vide infra*). At first, the role of kinase inhibitors on cholestasis is explained in further detail.

### Kinase inhibitors and cholestasis

For intrahepatic cholestasis, the formation of the bile itself from the hepatocytes is impaired. Bile formation is a complex process where many different transhepatic solute transporters are involved, most prominent at the basolateral site, the Na-taurocholate co-transporting polypeptide (NTCP), and the bile salt exporter (BSEP) and the multidrug resistance-associated proteins (MRPs) at the apical hepatocyte membrane (Anwer M.S., I.c). The plasma membrane localization of these transporters is a very dynamic process, which is regulated by posttranslational events, especially by kinases like protein kinase C (PKC), phosphoinositide 3-kinase (PI3K), AMP-activated protein kinase (AMPK) and mitogen-activated protein kinase (MAPK). It could be shown in various experiments that PKC inhibitors or PI3 Kinase inhibitors are useful preclinical tools for the treatment of cholestasis. (Anwer M.S., I.c.; Toledo et al. Arch Toxicol. 2017, 91:2391-2403; and Li et al Pharm. Res. 2017, 125, 105-113). These kinase inhibitors highly influence cell proliferation and the signaling of immune cells and act as immune suppressive agents.

### Kinase inhibitors and sepsis

As outlined above, sepsis is a serious, complex *systemic* immune reaction, triggered by an infection. The body relies on its immune system to counteract this infection. It is known in the art that kinase inhibitors, such as PKC and PI3 kinase inhibitors, are able to suppress an immune response in case of an inflammation. Therefore, it is suggested that an exaggerated inflammatory response can be treated with such compounds, also in case of sepsis (see, e.g., USH1168H, US5,558,969, WO93/16703, cited above). It has, however, to be realized on the one hand that certainly not the underlying infection itself is treated by such proposed treatments and on the other hand that for conditions associated with an infection, it is certainly not advisable to suppress the body's immune system. As already mentioned, immunocompromised patients have increased risk of infection by common pathogens and it is obvious that this highly developing risk of infection predisposes the patients to increased risk of sepsis and septic shock. Kinase inhibitors, such as PKC and PI3 kinase inhibitors, are also known to cause a higher incidence for various (additional) infections due to their immune suppressive properties. For these reasons, manufacturers of commercially available kinase inhibitors for the use in the treatment of different pathogen conditions, in particular kinds of cancer, indicate the immunosuppressive effect and explicitly warn against systemically administration of these inhibitors in presence of an infection or inflammation in the patient. See for example:
- EMEA report EMEA/H/C/753 of May 24, 2007, (Doc.Ref. EMEA/150964/2007), page 13, concerning PKC inhibitor ruboxistaurin (drug name Arxxant; indicated for diabetic retinopathy);
- In the Medscape site for healthcare professionals it is described for the PI3K inhibitor copanlisib (drug name Aliqopa; indicated for relapsed follicular lymphoma), that a withhold of the treatment in case of infection is necessary; The increased risk for infections (even sepsis itself) is described in detail by Kim et al. BJC, 2018, 118, 462-470.
- Medscape (I.c.) also prescribes a withhold of the treatment with PI3K inhibitor idelalisib (drug name Zydelig; indicated for 3 classes of lymphoma) in case of infection especially mentions **sepsis**; regulation: *"interrupt idelalisib with until infection has resolved".* This is also described in Zelenetz et al. Lancet Oncol. 2017, 18:297-311. They describe a five-fold higher risk of developing sepsis as adverse effect during idelalisib compared to placebo.
- Novartis Pharma GmbH (2017): Rydapt® 25 mg Weichkapseln, Fachinformation (professional information) status September 2017 provides the highlights of prescribing information on PKC inhibitor midostaurin (drug name RYDAPT; indicated for AML (acute myeloid leukemia). Pages 1, right column, and page 5, Table 2, indicate the adverse reactions of midostaurin; On page 6, last paragraph, it is stated "*Grade ≥ 3 adverse reactions reported in ≥ 5% were fatigue, **sepsis**, gastrointestinal hemorrhage, pneumonia, diarrhea*, *fibrile neutropenia*, .... *(Table 4).* On page 7 it is explicitly stated: *"Treatment discontinuation due to adverse reaction occurred in 21% of patients. The most frequent adverse reactions causing treatment discontinuation included infection.... Serious adverse reactions were reported in 68% of patients, most commonly (≥20%) due to infections and gastointestinal disorders.*" and *"On-treatment death unrelated to the underlying malignancy occurred in 16 patients (11%), most commonly from infection (**sepsis** or pneumonia), followed by cardiac events. Of the on-treatment deaths from disease progression, 4 were attributed to infection."* On page 8 adverse reaction occurring in ≥9% of patients suffering from sepsis is outlined.

From the afore-mentioned it is evident that kinase inhibitors are not to be administered systemically when sepsis is present. This is a teaching away, and, consequently, a skilled person would not contemplate to administer kinase inhibitors to treat cholestasis during a systemic infection, like sepsis.

In summary: As outlined above, the currently known treatments of septic cholestasis (also: sepsis-induced or sepsis-associated cholestasis) are treatments of the underlying systemic infection. For conditions with an underlying systemic infection it is not advisable to suppress inflammatory responses with systemically administered immune-suppressive kinase inhibitors, because the immune modulatory adverse effects could result in life-threatening conditions; especially when such drugs are administered systemically.

It is therefore an object of the invention to provide an effective treatment of septic cholestasis by avoiding, at least minimizing, adverse side effects. It is further an object of the invention to provide a direct treatment of septic cholestasis itself.

This object has been solved by the present invention and by treating septic cholestasis with compounds that lower the activity or inhibit protein kinase C (PKC), which finally regulate the transporters, responsible for bile formation. These compounds are targeted into the liver by a unique and selective delivery system.

The invention relates in its first aspect to inhibitors of the PKC signaling pathway for use in the treatment of septic cholestasis, wherein the inhibitors are targeted into the liver by a selective nanostructured delivery system, wherein the selective nanostructured delivery system comprises at least one carbohydrate targeting moiety and at least one polymer and/or at least one lipid and/or at least one virus-like particle. According to the invention, the inhibitors are preferably delivered into the parenchymal cells of the liver by the inventive selective nanostructured delivery system.

Bile formation is a complex process where many different transhepatic solute transporters are involved, most prominent the Na-taurocholate co-transporting polypeptide (NTCP), the bile salt exporter (BSEP) and the multidrug resistance-associated proteins (MRPs) (Anwer, I.c.). These transporters are located on the basal sites of the hepatocytes.

The plasma membrane localization of these transporters is a very dynamic process, which is regulated by posttranslational events, especially by kinases like protein kinases, such as protein kinase C (PKC), phosphoinositide 3-kinase (PI3K), AMP-activated protein kinase (AMPK) and mitogen-activated protein kinase (MAPK). It has been shown in various experiments that PKC or PI3K inhibitors are useful preclinical tools for the treatment of cholestasis. (Anwer, I.c.; Toledo et al., I.c.; Li et al., I.c.). These kinase inhibitors highly influence cell proliferation and signaling of immune cells and act as immune suppressive agents.

According to signaling pathways, the activity of PKC is highly dependent on the concentration of the regulatory molecules like diacyl glycerol (DAG) or calcium (Ca²⁺), e.g.:
▪ DAG concentration is mediated by enzymes like phospholipase C (PLC), which in turn is highly regulated by activation of various Gα_{q} coupled GPCRs, AKT- and MAP-kinases, growth factors and cannabinoid receptors.
▪ PLC activity is mainly regulated by PI3 kinases, which phosphorylate PIP₂ into PIP₃.
▪ Activated PLC cleaves PIP2 into IP3 and DAG. IP3 triggers Ca-release into the endoplasmatic reticulum (ER), which in turn activates PKC. The molecule DAG itself also contributes to the activation of the PKC.
▪ Therefore, PI3 kinase inhibitors, PLC inhibitors, DAG level reducing agents or any agents finally contributing to a reduced PKC are useful tools to treat septic cholestasis.

From the biochemical point of view, PI3 kinases produce the signaling molecule diacyl-glycerol (DAG), which in turn activates other protein kinases (e.g. PKC). Therefore, PI3 kinase inhibitors basically reduce DAG level, and hence also inhibit downstream, PKC. For this reason, agents capable for reducing DAG levels otherwise, are also useful in the treatment of septic cholestasis.

Accordingly, the term "inhibitor(s) of the PKC signaling pathway" of the invention relate to any substance(s) which influence the transmission and/or transduction of PKC mediated signals within the organism and cells. Such inhibitors especially comprise inhibitors of kinases which are involved in the PKC signaling pathway.

The term "inhibitor(s) of the PKC signaling pathway" further means any substance(s) which influence, preferably reduce or inhibit, the activity and/or express
ion and/or protein folding of PKC either directly or indirectly, e.g., via regulatory molecules upstream to the PKC pathway. In a preferred embodiment, the activity and/or expression and/or protein folding of PKC itself, PI3Kinase, DAG, PLC, AMPK, MAPK, AKT is reduced by the inventive "inhibitor(s) of the PKC signaling pathway". Inventive inhibitor(s) of the PKC signaling pathway can be understood as PKC activity reducing agents. According to the invention, the terms "inhibitors of the PKC signaling pathway", "PKC activity reducing agents" and "inhibitors of the PKC activity", "inhibitors of the PKC expression" and "inhibitors of the PKC folding" are used synonymously.

Such "inhibitor(s) of the PKC signaling pathway" can act either directly inhibit the abovementioned proteins / signaling molecules for example by:
- direct inhibition of PKC by PKC inhibitors, such as midostaurin, staurosporine, BIM I and other drugs; or by
- silencing of the protein biosynthesis of PKC or the respective proteins / signaling molecules by siRNA, miRNA, shRNA, modified oligo analouges or antisense constructs; and also by using other molecular biological methods known in the art, like CRISPR/Cas, TALEN, zincfinger nucleases or anti-sense oligo nucleotides.

Consequently, in a preferred embodiment, the inhibitors of the PKC signaling pathway for use in the treatment of septic cholestasis directly or indirectly inhibit or reduce the activity of PKC or any PKC subtype.

Direct inhibition or reduction of the activity of PKC according to the invention means influencing the activity by PKC inhibitors including nucleic acid constructs that silence the respective genes, preferably via RNAi. This can be accomplished by generally known methods, preferably by methods using constructs, such as siRNA, miRNA, shRNA, RNAse H, modified oligomers, like morpholinos. According to the known biochemical approaches, respective nucleic acid constructs are designed and either directly bound to the carbohydrate targeting moiety by covalent binding, or by encapsulation in nanocarriers bearing the carbohydrate targeting moiety.

The indirect inhibition or reduction of the activity of PKC according to the invention is preferably accomplished by inhibition or reduction of pathways and/or signaling molecules, necessary for PKC activity. PKC activity is driven by various factors: PI3 kinase inhibitors, PLC inhibitors, DAG level reducing agents or any agents finally contributing to a reduced PKC are useful tools to treat septic cholestasis. Therefore, all kinds of agents (e.g. PI3 kinase Inhibitors, PLC inhibitors), capable to influence the PKC signaling pathway by reducing PKC activity are inhibitors according to the invention and useful tools for the treatment of septic cholestasis. This can again be achieved either by small molecule inhibitors, such as PI3 kinase inhibitors described herein, and/or PLC inhibitors like U-73122, D609, manalide, edelfosin or respective nucleic acid constructs.

Direct and indirect inhibition or reduction of the PKC activity according to the invention has also to be understood and comprises any influence on the expression of PKC genes including PKC subtype genes, their transcription and/or translation and/or protein folding resulting in a decreased and/or no gene product and/or PKC protein. Such an influence has to be understood as decreasing (reduction) or preventing, turning off, switching off (inhibition) the PKC expression, and thus PKC activity.

Presently, PKC inhibitors are predominantly used in cancer therapy and in the treatment of autoimmune diseases. Currently two PKC inhibitors are available as proofed drugs, namely Rydapt® (midostaurin) and Arxxant® (ruboxistaurin).

Further known drugs relate to idelasilib and copanlisib. In the information site for professionals for both compounds (Medscape references: referred to / cited above) it is due to their immunosuppressive properties strictly contraindicated in case of serious infections including sepsis and it is advised to discontinue the therapy during that time.

Until now, no specific treatment of septic cholestasis in the clinics is available and treatment with any of the above-mentioned agents systemically administered would be too risky due to the fragile immune status during underlying sepsis and the therapeutic dose in the liver required to elicit any positive effects on septic cholestasis.

The present invention therefore provides for the first time a specific treatment of septic cholestasis itself by avoiding, at least reducing, dangerous, systemic immunosuppressive effects on the body of a patient. This is achieved according to the invention by a selective targeting of therapeutic agents to the side of action, i.e. the liver parenchymal cells. The inventive nanostructured delivery system provides an active hepatocyte targeting to treat septic cholestasis, which is accomplished by carbohydrate-derived targeting moieties, which are selectively recognized by special lectins, present on liver tissue. According to the invention, the systemic circulation and also the required therapeutic dose of these therapeutically active agents can be highly reduced compared to systemic administration of PKC inhibitors in the treatment of pathological conditions.

According to the invention, the inhibitors are administered, e.g. by injection, and selectively delivered, by the inventive nanostructured delivery system, to their site of action, i.e. the liver, to treat septic cholestasis. In this way, the side effects (immunosuppression) associated with a systemic treatment of infections with kinase inhibitors described above without delivering the inhibitor to the desired side of action are reduced, preferably avoided. Additionally, the required dose of the inhibitor with a nanostructured delivery system is greatly reduced compared to the dose of an inhibitor without the liver targeted nanostructured delivery system. The present invention provides for the first time a treatment of septic cholestasis itself.

For treating septic cholestasis as a condition with an underlying systemic infection, i.e. sepsis, it is not advisable to suppress the immune system. The present invention therefore describes a carbohydrate-driven selective delivery system for the targeted transport of inhibitors of the PKC signaling pathway, into the liver with a negligible systemic immune suppressive effect, because they selectively act in the liver where they specifically modify only the bile excretion and are capable to resolve the cholestasis. By this way, the agents of this invention, i.e. inhibitors of the PKC signaling pathway / inhibitors of the PKC activity, are administered in much lower doses compared to an untargeted counterpart, are selectively transported to the exclusively to the place of action and therefore are suitable for the treatment of septic cholestasis. Accordingly, the present invention represents a highly effective way for treating septic cholestasis systemic, adverse effects which generally occur when kinase inhibitors are administered in the state of the art treatments of infections, are greatly reduced.

The term "agent(s)" or "therapeutic agent(s)" according to the invention means inhibitors of the PKC signalling pathway; the term "agent" or "agents" is further used synonymously to the terms "anti-cholestatic agents" and "choleretic agents" as well as "drug" or "drugs". Furthermore, the terms "agent(s)" and "drug(s) are used synonymously according to the invention.

If the nanostructured delivery system according to the invention comprises at least one polymer, it is referred to herein as "nanoparticles"; if it comprises at least one lipid, it is referred to herein as a "liposome." If the nanostructured delivery system according to the invention comprises both polymers and lipids, it is also referred to herein as "nanoparticle" or as "liposome". If the nanostructured delivery system according to the invention comprises at least one polymer and at least one nucleic acid construct, it is also referred to herein as "polyplex". According to the invention, nanoparticle, liposom, virus-like particle as well as polyplexes, lipoplexes and peptoplexes relate to the nanostructured delivery system.

Nanoparticles may be constructed of a plurality of molecules. These nanoparticles may consist of polymers wherein these polymers are characterized by the fact that certain units (monomers) are repeating units. The polymers are covalently bonded to one another by the chemical reaction of these monomers (polymerization). If some of these polymers have hydrophobic properties, they may form nanoscale structures (e.g., nanoparticles, micelles, vesicles) in an aqueous environment. Due to their hydrophobic properties, lipids may also be used to form nanoparticles (micelles, liposomes).

If the nanostructured delivery system according to the invention comprises at least one positively charged polymer, which is complexed with negatively charged genetic material, it is referred to herein as "polyplex"; if it comprises at least one positively charged lipid, and negatively charged genetic material, it is referred to herein as a "lipoplex" and if it comprises at least one positively charged peptide, and negatively charged genetic material, it is referred to herein as a "peptoplex".

According to the invention, the terms "carbohydrate targeting moiety", "carbohydrate-based targeting moiety", and "carbohydrate derived targeting moiety" have the same meaning and can be used synonymously. A carbohydrate targeting moiety (CTM) according to the invention means a chemical structure, which is recognized by special surface molecules (e.g. lectins), preferably the ASGP receptor, and induces internalization of a construct, agent, nanostructured delivery system, i.e. the nanostuctured delivery system according to the invention, into the cells, tissue or organs, preferably liver, where these surface molecules are expressed. The CTM can be either monovalent or multivalent, depending on the labelling density on the surface of the agent or agent construct or polymer. In multivalent setup, there is a core molecule, which bears at least one single unit (preferably derivatives of *N*-acetyl-galactosamine (GalNAc), galactose mannose, and glucosamine). It can be either a repetition of the same unit or a mix of different units. CTMs with a higher MW and multiple repetition units (e.g. pullulan or arabinogalactane) could form a nanostructured carrier system by themselves, but can also be attached to the nanostructured carrier.

In a preferred embodiment the carbohydrate targeting moiety is selected from the group consisting of of *N*-acetyl-galactosamine (GalNAc), galactose, lactose, mannose, glucosamine, asialofetuin, pullulan, arabinogalactan, glycorrhizin, glycorrhetinic acid and derivatives thereof. Preferably, the carbohydrate targeting moiety is recognized by an ASGPR recognition moiety.

Carbohydrate targeting moieties or carbohydrate liver or hepatocyte recognition moieties according to the invention comprise classical monovalent ligands of ASGPR like galactose, glucosamine, *N*-acetyl-galactosamine (GalNAc) oligosachharide constructs, or multivalent constructs bearing these recognition units. Further preferred are unclassified surface recognition moieties, which could be addressed with fuconic acid, lactobionic acid, mannose, fibronectin, transferrin, asialofetuin, glycyrrhetinic acid, lithocholytaurin, sterylglycosides lipoproteins or specific ASGPR-antibodies.

Consequently, in a preferred embodiment of the invention the carbohydrate targeting moiety binds to a recognizing unit, located on the liver.

In a further preferred embodiment, this recognizing unit or target unit is a receptor belonging to the family of lectins, preferably a lectin, more preferably the asialoglycoprotein receptor (ASGPR), aka Ashwell-Morell receptor.

The most prominent representative of hepatocyte-specific lectins is the asialoglycoprotein receptor (ASGPR). ASPGR is a liver-specific membrane-bound receptor involved in the endocytosis of carbohydrate-containing glycoproteins. This receptor acts like a "lock" on the direct way into the hepatocytes and recent studies thoroughly investigated this lock for its properties and possible keys (Sanhueza C.A. et al., Efficient liver targeting by polyvalent display of a compact ligand for the asialoglycoprotein receptor; JACS, 2017; 139: 3528-3536). After binding of appropriate ligands (keys), the receptor and the whole ligand construct become internalized into the hepatocyte, preferably by clathrine-mediated endocytosis.

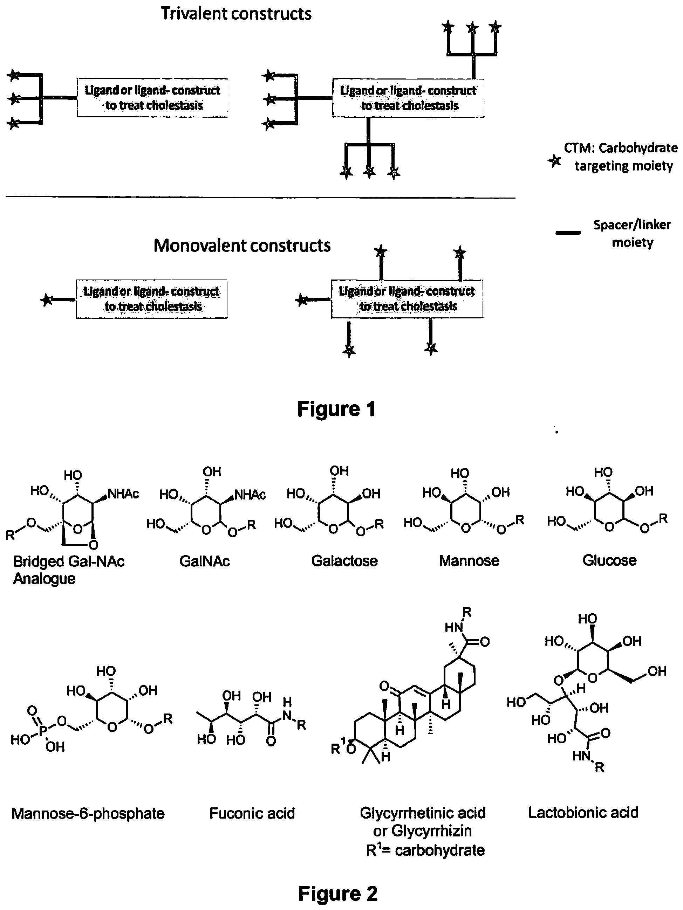

By X-ray crystallography it has been shown that ASGPR harbors a shallow binding cavity, which is best targeted by multivalent ligand constructs. Such multivalent structures are state of the art and can be set up in different ways and with different substructures (see fig. 2). Sanhueza et al. *I.c*. give an overview of possible setups for the multivalent ligands, but basically any molecule with and connection point (connection to agent/agent construct/ polymer) and three further connecting points to connect the smallest unit of the carbohydrate targeting unit might be suitable (see fig. 1). Depending on the overall surface density of the targeting moiety, different setups might be best suited. If the surface labelling of a nanoparticle is high enough, monovalent ASPGR ligands might also be suitable for an appropriate targeting. From case to case, the most efficient and synthetically feasible targeting has to be investigated, for example in a suitable model for tissue endocytosis like a chip-based microfluidic model (exemplified in Example 8).

In an especially preferred embodiment, the inhibitors of the PKC signaling pathway for use in the treatment of septic cholestasis are selected from the group consisting of PKC inhibitors, PI3 kinase inhibitors, MAPK inhibitors, PLC inhibitors, DAG level reducing agents, siRNA, miRNA, shRNA, modified oligo analogues (e.g. morpholinos), antisense constructs and RNAse H.

The inhibitors siRNA, miRNA, shRNA, modified oligo analogues (e.g. morpholinos), antisense constructs and RNAse H according to the invention relate to oligonucleotide constructs capable to silence the respective genes (e.g. silence of PKC gene, PI3 Kinase gene, MAPK gene, PLC gene) which can be constructed by gene silencing techniques well known in the art. These inhibitors can also be designated as PKC siRNA, PKC shRNA, PKC miRNA, PI3 siRNA, PI3 shRNA, PI3 miRNA.

According to the invention, inhibition of PKC activity can also be achieved with appropriate gene editing methods like CRISPR/Cas, TALEN, zincfinger nucleases.

In a preferred embodiment, the inhibitors of the PKC signaling pathway for use in the treatment of septic cholestasis are PKC inhibitors selected from the group consisting of bisindolylmaleimides, staurosporine, midostaurin, UCN-01, sotrastaurin, enzastaurin, ruboxistaurine, tivantinib, enzastaurin, Gö 6983, K252a, ANA-12, lestaurtinib, stauprimide, CEP-701, Arcyriaflavin a, chelerythrine chloride, and Bisindolylmaleimids I-XII aka BIM I-XII.

In a preferred embodiment, the inhibitors of the PKC signaling pathway for use in the treatment of septic cholestasis are PI3 kinase inhibitors selected from the group consisting of copanlisib, idelalisib, wortmannin derivatives, bryostain derivatives, taselisib, omipalisib, AS605240, GSK1059615, buparlisib, alpelisib, pictilisib, serabilisib, dactolisib, dihydrosphingosine, calphostin C and melittin. Preferred inhibitors of the invention also comprise novel research compounds exhibiting PI3 kinase inhibitory effects.

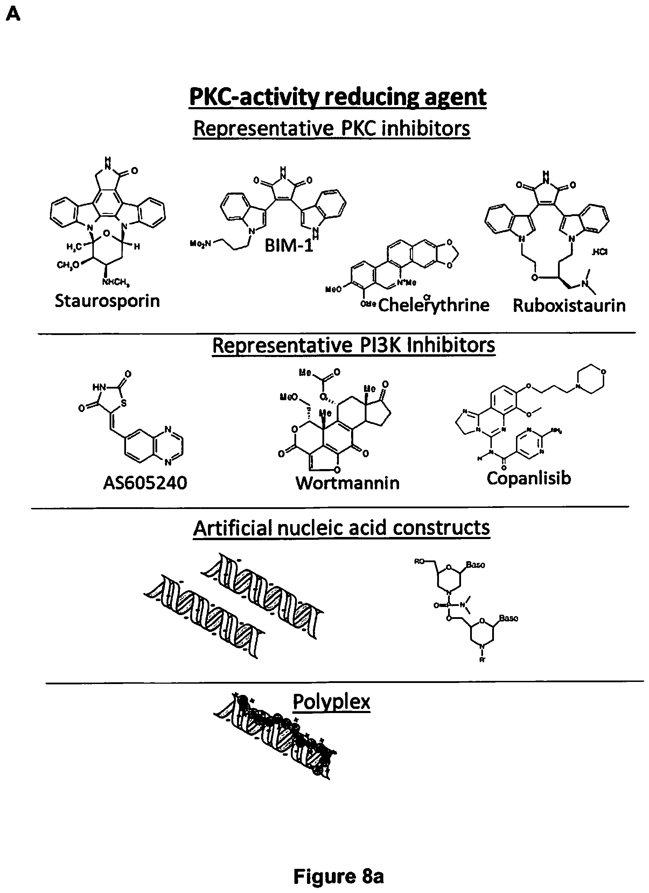

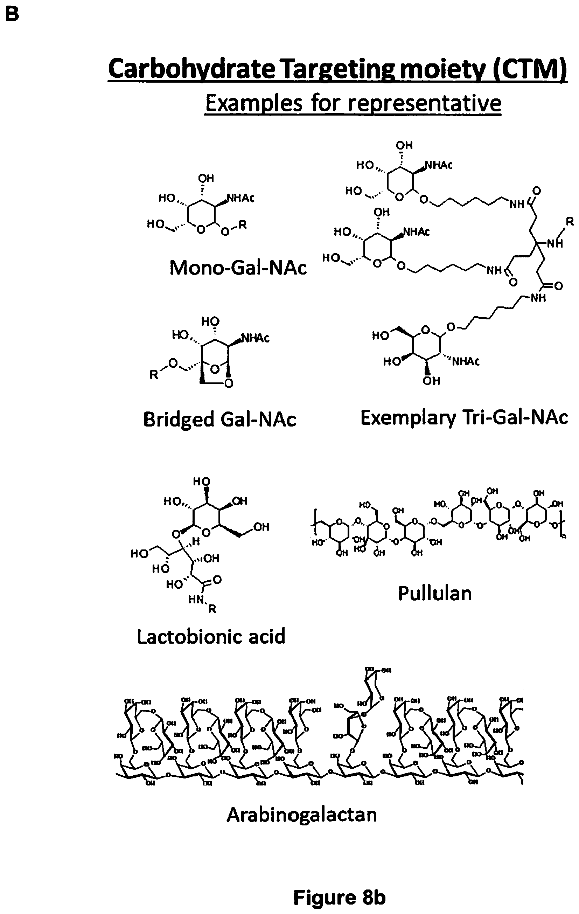

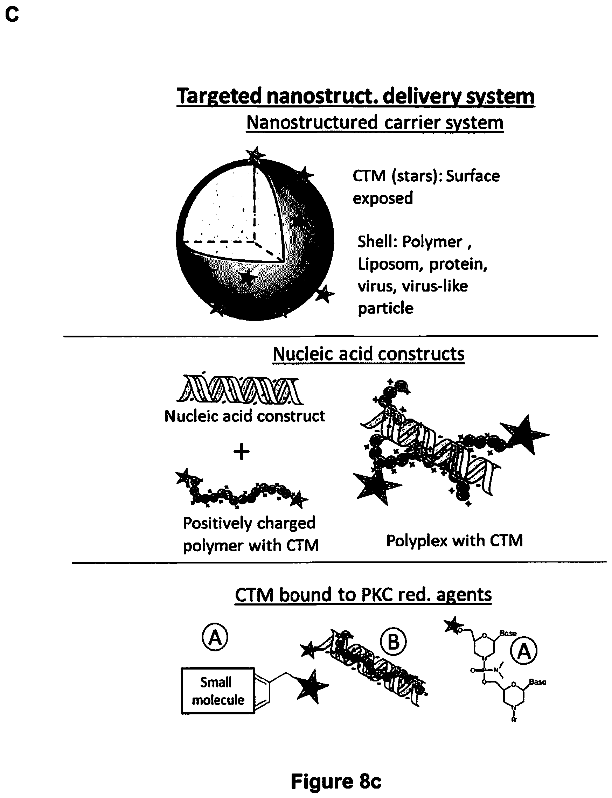

The agents of the invention, i.e. inhibitors of the PKC signaling pathway, can either be directly coupled to a spacer or linker comprising an aliphatic, heretoaliphytic, aromatic, heteroaromatic, linear, branched or cyclic assembly of atoms, and/or a carbohydrate targeting moiety or coupled to a suitable carrier like nanoparticles, liposomes or virus-like particle, in which these agents are encapsulated or trapped (see, for example, **Figures 1**, **8**).

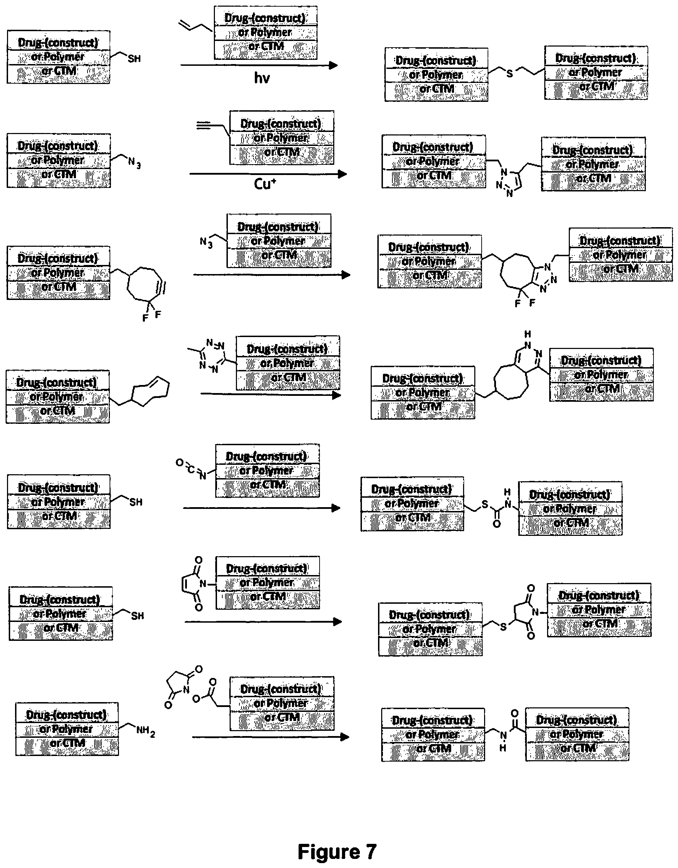

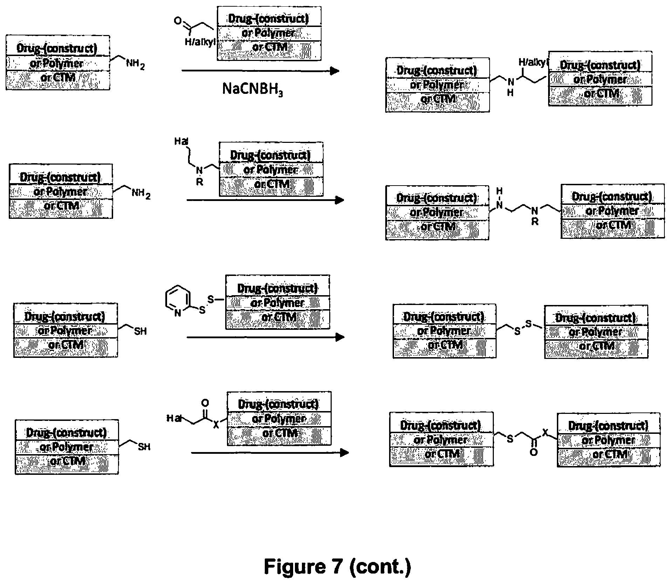

The inventive carbohydrate targeting moiety as selective liver targeting moiety can be attached to the agent of the invention (i.e. inhibitor direct or suitable carrier) by regular chemical coupling reactions which are well-known in the state of the art, preferably by activated carboxylic acid derivatives (e.g. anhydrites, acyl halides, active esters) and subsequent coupling to amines, by photo-induced thiol-ene click reaction, by Michael addition (1,4-addition), cycloaddition reactions, Huisgen reaction (e.g.1,3-cycloaddition of alkynes to azides), Diels-Alder reaction (e.g. trans-cyclooctene coupling to tetrazine derivatives), maleimide-thiole reaction, isocyanate-, isothiocyanate coupling, carbodiimide coupling, chloroacetamide coupling. Alternatively, reactive carbonyl compounds, preferably ketones, aldehydes acetals or hemiacetals with amines to form a Schiff-base, which can be reduced to a corresponding amine, can be used according to the invention (see, e.g., **Figure 7**).

The term "nanostructured delivery system" according to the invention is characterized by at least one carbohydrate targeting moiety and at least one polymer and/or at least one lipid and/or at least one virus-like particle, which delivers the therapeutic agent, i.e. inhibitors of the invention, into a target tissue, comprising contacting a target tissue with said nanostructured delivery system.

The at least one carbohydrate targeting moiety as a targeting unit triggers the active and selective transport of the nanostructured delivery system into the target tissue.

The at least one carbohydrate targeting moiety further triggers the uptake of the nanostructured delivery system into the cells of the target tissue by interacting with the cell surface and accumulating the nanostructured delivery system on the cell surface.

The term "nanostructured delivery system" according to the invention also relates to polyplexes, which have to be understood as complexes between a negatively charged nucleic acid construct ligated to a positively charged polymer. The terms "nanostructured carrier system" and "nanostructured delivery system" are used synonymously according to the invention.

The inventive nanostructured delivery system comprises a combination of a nanostructured carrier and a carbohydrate targeting moiety. The nanostructured delivery system comprises at least one polymer and/or at least one lipid or virus-like particle and is capable of carrying an active ingredient - according to the invention a PKC activity inhibiting or reducing agent (inhibitor of the PKC signaling pathway) like a vehicle. These nanostructured systems can be detected and characterized by methods known in the art, such as DLS, AUC, AF4, DSC, ITC, XRD, SANS, SAXS or special microscopic methods like SEM, STEM or kryo-TEM, AFM. The shape can be preferably, but not limited thereto, either spherical, oval, rod-like, barrel-like, disc-like or polyhedral. The size preferably varies from 1 nm to 800 nm.

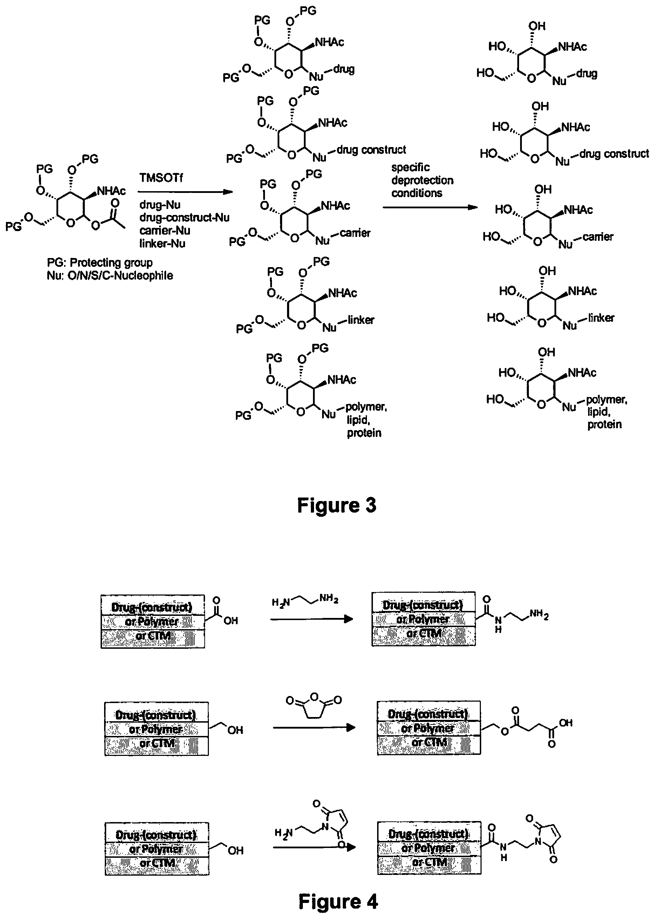

In a preferred embodiment, the at least one polymer, lipid, virus-like particle and/or active agent contains functional groups, which allow chemical modifications and the attachment of a carbohydrate targeting moiety (CTM) (see, e.g., Fig. 4 and Fig. 7). The polymer might be organic or inorganic and fixes the therapeutic agent like a vehicle. Inorganic particles might be functionalized by silanization with functionalized silans like aminopropy-trimethylsilan (APTES), which introduces amine functions to oxides.

In a preferred embodiment of the invention, the at least one polymer is selected from the group consisting of polyesters, polyacrylates, polystyrene derivatives, polyamides, polyurethanes, polyacrylonitriles, polytetrafluoroethylenes, silicones, silica particles, cerium oxid aluminum oxide or apatite particles, polyethylene glycols, polyethylene oxides and polyoxazolines and their copolymers, preferably in a variety of compositions such as random, gradient, alternating, block, graft or star copolymers. More preferred the at least one polymer is an organic, inorganic, hydrophobic, hydrophilic, amphiphilic, anionic and/or cationic polymer.

The polymer is even more preferably selected from the group consisting of PLGA, PLA, PCL, PGA, PDMAEMA, PMMA, PMAA, PEI, PEtOx, PEG, HPMA, APMA, PVP, hydrolyzed PVP, polysaccharides, such as arabinogalactan, chitosan, pullulan, alginate, cellulose or starch derivatives. Polymer according to the invention also comprise inorganic polymers, which can form porous particles, capable for trapping / encapsulating of active ingredients, preferably silica-based, alumina-based, titanium oxide-based, cerium oxide-based, carbon-based, zeolite-based, or apatite-based.

In a preferred embodiment of the invention, the at least one lipid is selected from the group consisting of saturated and unsaturated fatty acids, cholesterol derivatives, phospholipids, sphingolipids, lipoproteins and glycolipids.

The at least one polymer and/or at least one lipid according to the invention is/are preferably a biocompatible polymer and/or lipid.

The nanostructured delivery system preferably comprises a virus-like particle, e.g. a protein or protein shell. Such virus-like particle preferably comprises a protein shell, preferably, but not limited thereto, derived from the following viruses: Bacteriophage MS2, Bacteriophage Qβ, Enterobacteria phage P22, Cowpea mosaic virus (CPMV) Cowpea Chlorotic Mottle Virus (CCMV), hepatitis B virus carries (HBVc), Adeno associated virus (AAV). The proteins are obtained by transfection of the respective virus-genetic material into a suitable expression system like Saccharomyces cerevisiae by methods well-known in the art.

Consequently, in a preferred embodiment of the invention, the at least one virus-like particle is derived from a virus selected from the group consisting of Bacteriophage MS2, Bacteriophage Qβ, Enterobacteria phage P22, Cowpea mosaic virus (CPMV) Cowpea Chlorotic Mottle Virus (CCMV), hepatitis B virus carries (HBVc) and Adeno associated virus (AAV).

The invention will be illustrated in more detail with reference to the Figures, which not have to be understood to limit the scope of the invention.
**Figure 1** shows a schematic representation of different inventive constructs; monovalent and multivalent (at present trivalent) CTM on ligand or ligand construct (also nanostructured carrier).
**Figure 2** shows examples for lectine-binding moieties useful for hepatocyte targeting "R" represent a possible connection point for the delivery system (polymer, virus-like particle, lipid or a genetic construct).
**Figure 3** demonstrates a general synthetic approach for the synthesis / attachment of lectin-binding carbohydrate moieties to agents, agent constructs, carrier polymers, virus-like particles or linkers.
**Figure 4** demonstrates exemplified methods to introduce and/or change functional groups for the attachment of an agent/agent construct, polymers and targeting moieties.
**Figure 5** demonstrates an exemplified synthesis for GalNAc PLGA (here shown with a GalNAc monomer). The same procedure can be adopted for the synthesis of a trivalent GalNAc construct.
**Figure 6** shows strategies for the direct coupling of nucleic acid material to a CTM. Fig. 6 **A**: 3'EndLabeling strategy mainly for DNA-like constructs; Fig. 6 **B**: 5'EndLabeling strategy for DNA, RNA or modified nucleotides.
**Figure 7** demonstrates examples for the connection strategies between agent, or agent construct with polymer and/or targeting moiety.
**Figure 8** shows an exemplary building block to generate/prepare various different nanostructured delivery systems, useful for the treatment of septic cholestasis. Fig. 8a shows a variety of potential compounds, which reduce PKC activity (**A**); Fig. 8b shows some carbohydrate targeting moieties (CTMs) (**B**); Fig. 8c shows examples for targeted nanostructured delivery systems (**C**).
**Figure 9** shows the synthetic route to carboxy- and amine-functionalized GalNAc derivatives. For the design of monomeric carbohydrate-based targeting units, the synthetic route to GalNAc derivatives is shown; basically this scheme can be adopted to other carbohydrate derivatives. The carboxy-terminated CTM can be coupled to any amine-terminated carrier, polymer, lipid, protein or drug, whereas the amine-terminated CTM can be coupled to any carrier, polymer, lipid, protein or drug via regular peptide coupling, known for a skilled person (e.g. EDC/NHS).
**Figure 10** shows a scheme for the synthesis of a trivalent Gal-NAc construct with maleimide linker to couple the construct to a thiol group.
**Figure 11** shows a scheme for the synthesis of an amino-terminated trivalent GalNAc construct.
**Figure 12** shows the coupling of amino-terminated GalNAc to the terminal carboxylic acids from PLGA.
**Figure 13** demonstrates methods for the preparation of nanoparticles by emulsion, double emulsion and nanoprecipitation. Fig. 13 **A**: Emulsion and double emulsion; Fig. 13 **B**: Nanoprecipitation.
**Figure 14** shows Toxicity of targeted nanoparticles vs free drug (BIM-1) in HepG2.

The carbohydrate moiety triggered endocytosis according to the invention can be adopted for the tissue specific transport of the agent. For this, the agent of interest is coupled with a linker/spacer, which contains the ASGPR-specific recognition ligand or ligand construct. According to the invention, the inhibitors of the PKC signaling pathway are coupled either directly or with a spacer comprising the ASGRP-specific recognition ligand as shown in **Fig. 1** or ligand construct to the polymer or a nanostructured delivery system (i.e. polymer particle).

The ASPGR-specific recognition ligand might be GalNAc or another liver-specific lectin recognition ligand as shown in **Figure 2**. These recognition ligands are responsible for the targeted delivery and cell / tissue / organ specificity of the agent, agent construct or carrier. As outlined in **Figure 2**, such recognition ligands preferably are derivatives of carbohydrates. They are useful for hepatocyte targeting. The shown molecules are preferred for the construction of a CTM, but also larger molecules like pullulan- or arabinogalactan derivatives (as shown in **Fig. 8**) could be used.

To couple the ASPGR-specific carbohydrate based recognition moiety to a drug (agent), drug construct (agent construct) or carrier, different approaches can be applied. Depending on the functional groups present in the respective drug, drug construct or inventive nanostructured delivery system and the respective recognition ligand, the most suitable method has to be evaluated. In case of carbohydrate derivatives, suitable leaving groups (e.g. acetates) are preferably further activated by TMSOTf or HBr and subsequently substituted by various nucleophiles, like alcohols, amines, thiols or C-nucleophiles as demonstrated in **Figure 3**.

In case the direct coupling is difficult due to a lack of suitable connection points, suitable functional groups are preferably introduced according to generally known functional group interconversion methods as shown in **Figure 4** to link the agent/agent construct, or nanostructured delivery system to the carbohydrate targeting moiety. **Fig. 4** shows the interconversion of carboxylic acid to amine, alcohol to carboxylic acid and alcohol to maleimide. The carboxylic acids are suitable for coupling with amines and vice versa, whereas maleimides can be coupled to thiols.

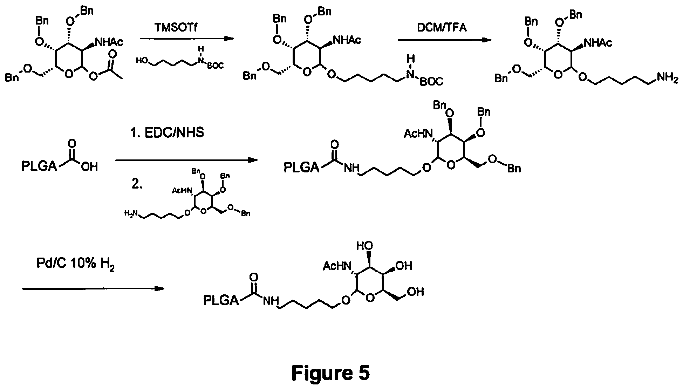

The carbohydrate targeting moiety (e.g., ASPGR-recognition moiety) can either be attached directly, or via additional spacers to increase the distance between targeting moiety and the agent/agent construct, polymer and/or delivery system. For the synthesis of Gal-NAc PLGA, useful as nanocarrier, an exemplified synthesis is shown in **Figure 5**. Further disclosure is given in Example 3. GalNAc labelled PLGA (**Fig**. **5** and Example 3) is useful for the encapsulation of the PKC-inhibiting agent preferably by nanoprecipitation as, for example, described in Example 4, emulsion or double emulsion.

Alternatively, the carbohydrate targeting moiety (here GalNAc) can be coupled to the final particle (nanostructured delivery system) after encapsulation of the inhibitor of the invention. In this case, the inhibitor of the PKC signaling pathway is encapsulated accordingly. After the preparation of the nanoparticle, the functional groups in the polymer are activated and coupled to the CTM analogously to the coupling as shown in **Fig. 3**. Depending on the functional groups on the polymer and the drug used, different coupling strategies can be applied. Such coupling strategies are well-known in the art; preferred coupling strategies usable according to the invention are shown in **Fig. 7**.

This approach can be adopted to small molecules, nucleic acid constructs, like si-RNA, or inventive carriers, such as liposomes or nanoparticles, either organic or inorganic. Methods to be used are known in the art and described, for examples, in Huang, Mol. Ther. Nucl. Acids, 2017, Preclinical and Clinical Advances of GalNAc-decorated Nucleic Acid Therapeutics Molecular Therapy: Nucleic Acids Vol. 6, 2017 p.116 or in Ahmed and Narain, Carbohydrate-based materials for targeted delivery of drugs and genes to the liver, Nanomedicine (Lond.) (2015) 10(14), 2263-2288.

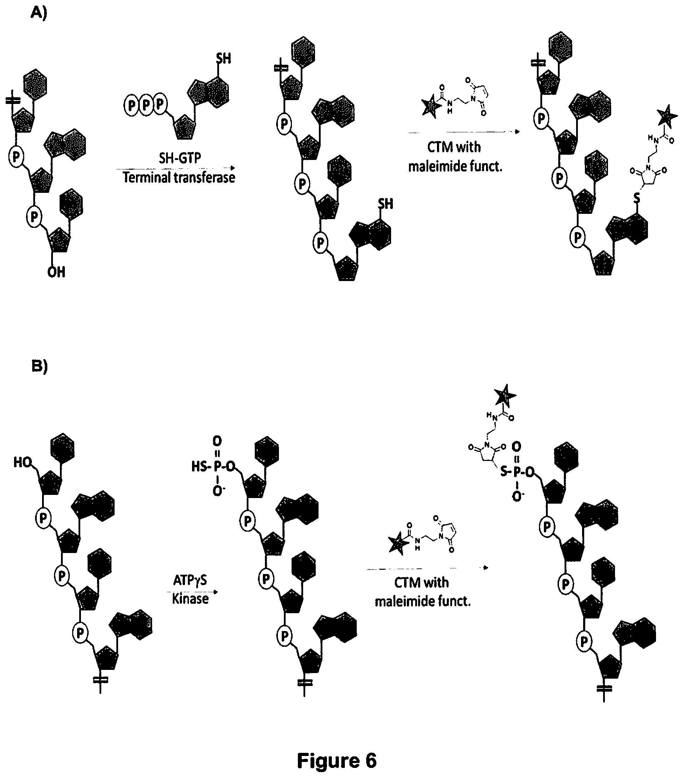

The carbohydrate targeting moiety (CTM) according to the invention is preferably attached to the polymeric moiety (polymer or virus like particle) of the nanostructured delivery system, but can also be directly attached to the inhibitor of the PKC signaling pathway before the formation of the nanostructured delivery system. For example, a carbohydrate targeting moiety comprising a maleimide functional group is attached to the nucleic acid construct by known labelling methods like 3'or 5'EndTAG™ For both methods, the preferable functional group at the CTM is the maleimide, which can be generated as shown in **Figure 3**. The two EndTAG coupling strategies to selectively couple nucleic acid constructs to the carbohydrate targeting moiety are shown in **Figure 7**. Strategies for the coupling of nucleic acid material to a CTM are shown in **Figure 6. Fig. 6A** shows 3'EndLabeling strategy mainly for DNA-like constructs; Fig. 6B shows 5'EndLabeling strategy for DNA, RNA or modified nucleotides. The targeted nucleic acid constructs can be used to form polyplexes with polymers (organic or inorganic) in order to generate a nanostructured delivery system.

**Figure 7** demonstrates examples for binding strategies for agent/agent constructs with the polymer or targeting moiety according to the invention. The inventive carbohydrate driven targeting moiety selective liver targeting moiety can be attached to the agent or agent construct of the invention by regular chemical coupling reactions as referred to above. For the coupling reaction, all the reactions, well known for a skilled chemist can be applied. In a preferred embodiment, reactive carbonyl compounds, preferably ketones, aldehydes acetals or hemiacetals with amines to form a Schiff-base, which can be reduced to a corresponding amine are used as shown in **Fig. 7**.

The carbohydrate targeting moiety comprises a chemical moiety, which is recognized by certain recognition unit, preferably a lectin, on the surface of the target tissue, preferably the liver. A preferred lectin for the recognition comprises the ASGPR and GalNAc constructs as carbohydrate targeting moiety. Furthermore, galactose-terminated glycoproteins, arabinoglycans, pullulans and sitosterol glycosides (aka sitoG) are useful as lectin recognition constructs. In **Fig. 8B** some representative carbohydrate targeting moieties are shown.

**Figure 8** shows an exemplary building block to prepare various different nanostructured delivery systems according to the invention, which are useful for the inventive treatment of septic cholestasis.. **Figure 8a** shows different PKC-activity reducing agents are shown which might be used in the building block. These PKC-activity reducing agents as well as small molecules and also nucleic acid constructs can be used according to the invention.. **Figure 8a** shows different preferred carbohydrate targeting moieties (CTM) which can be used according to the invention are shown. Monovalent, trivalent and multivalent. "R" represents the connection points to the agent/agent construct or polymer. Possible chemical bindings are shown in **Fig. 6**. The setup of the trivalent construct is only exemplary and the chains might also contain PEGs, amides, triazols or other moieties and the length of the chains can vary between 2 and 30 atoms **Figure 8c** shows different delivery systems are shown that carry the carbohydrate targeting moiety (shown as asterisks). On top, a polymer (organic or inorganic), lipid, virus-like particle is shown, which can act as a vehicle for the targeted drug delivery. Basically, the CTM can be linked to small molecules, nucleic acid constructs and also polyplexes between nucleic acid construct and a positively charged polymer. Such positively charged polymers can also be labelled with a carbohydrate targeting moiety (CTM) of the invention and hence also form after ligation to nucleic acid constructs, a targeted nanostructured delivery system by themselves. Such targeted nanostructured delivery systems are preferably formed, if the CTM is directly bound to the inventive inhibitors of the PKC signaling pathway (preferably nucleic acid constructs) and these constructs form a nanostructured delivery system (with or without helper polymer).

The invention is further demonstrated below on the basis of Examples, although it is not limited thereto.

### Examples

### Example 1: Synthesis of precursors for the synthesis of CTM (here GalNAc constrcts)

For the synthesis of precursors, 1 mmol of a fully acylated carbohydrate is activated with TMSOTf in DCM for 16h at rt. In the presence of the respective alcohol, the chain-functionalized carbohydrates are obtained with 80-90% yield. The CBZ-amine or benzylester moiety is deprotected by catalytic hydrogenation with 20% Pd/C. The monomeric carbohydrate moieties are coupled with simple peptide chemistry to a small molecule. Finally, the acetyl groups are cleaved off with 2mM TFA in DCM. Here with a copanlisib derivative (copanlisib-NH₂) as an PI3 Kinase inhibitor.

### Example 2: Synthesis of trivalent Gal-NAC constructs

### A: Maleimide functionalized trivalent Gal-NAc for direct coupling to nucleic acid constructs via introduced SH groups. (EndTAG® Labeling)

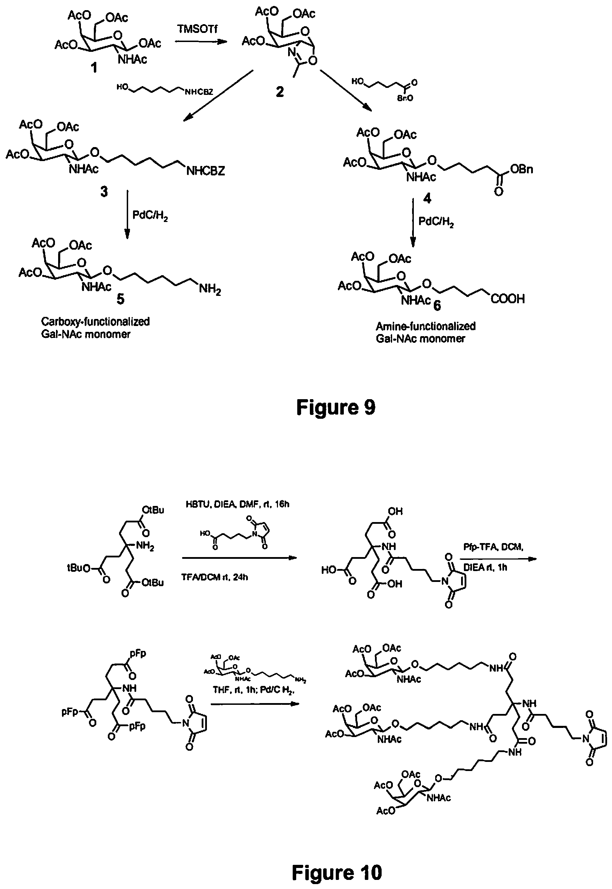

Aminotriester (1g) is dissolved in 10 mL of DMF, 5 eq. of HBTU and DIEA are added at rt. Under nitrogen, 1 eq. 5-maleimido valeric acid is added and stirred for 24h at rt. The reaction mixture is poured into 250 mL of 10% NaHCO₃ and extracted three times with ethyl acetate. The combined organic phases are evaporated to dryness and dissolved and stirred for 24 h in 25 mL DCM, containing 1M TFA. After evaporation of the solvents, the residue is dissolved in 300 mL of acetone and 3.5 eq of NaOMe are added. The precipitating compound is filtered off, dried and used without further purification. 1g of the tri-sodium salt is dissolved in water, acidified to pH2 and extracted 3 times with chloroform. The pooled organic phases are evaporated to a final volume of 50 mL and to the resulting tri-acid 5 eq. of Pfp-TFA and 20 eq. of DIEA are added and the reaction mixture is stirred for 2h. After the reaction, the mixture is poured into 500 mL of water, extracted 3 times with 200 mL of EtOAc, washed with brine, separated, dried over MgSO₄ and evaporated to dryness. The resulting gum is crystallized from hexane / ethyl acetate to give a beige solid.
Tris-Pfp ester is dissolved in THF and 5 eq. of the amino-GalNac monomer are added and stirred for 20 min. The reaction mixture is filtered off and evaporated to dryness. The resulting oil is subjected to column chromatography using CHCl₃ / MeOH 9:1 to yield the final trivalent GalNAc maleimide construct (detection with KMnO₄ or conc. H₂SO₄). The synthesis scheme is shown in Fig. 10.

### Direct coupling of genetic material-based inhibitors to a carbohydrate targeting moiety (EndTag®)

According to vectorlabs ®, 1µg PKC-siRNA (custom made by JenaBioscience) is incubated with T4 polynucleotide kinase and ATPγS in reaction buffer for 30 min at 37°C. The reaction is purified with a ThermoFischer RNA purification kit and stored carefully, as it is necessary for RNA. (low temperature, sterile and RNAse free!). The activated siRNA is then suspended in 50µL of PBS buffer and 1µg of trivalent Gal-NAc maleimide is added and shaken for 30 min at 65°C. The final construct is purified again under sterile conditions with a ThermoFischer RNA purification kit.

### B: Amine functionalized trivalent Gal-NAc for coupling to carboxylic acid derivatives (here PLGA)

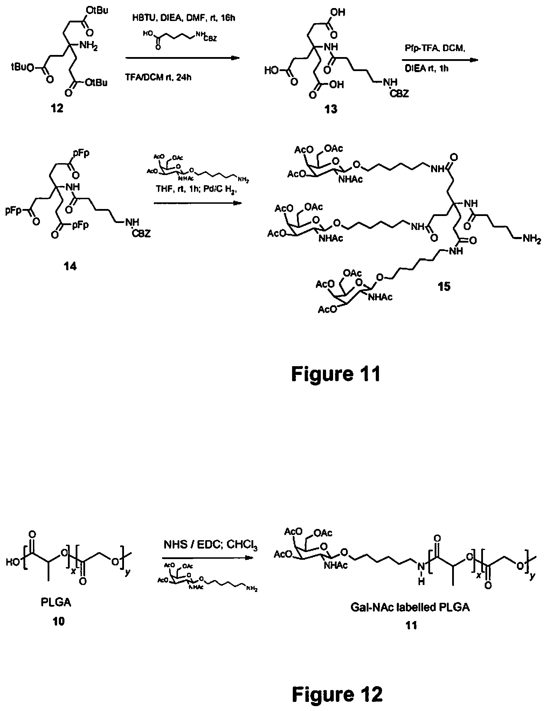

Aminotriester (1g) is dissolved in 10 mL of DMF, 5 eq. of HBTU and DIEA are added at rt. Under nitrogen, 1 eq. CBZ-5-amino valeric acid is added and stirred for 24h at rt. The reaction mixture is poured into 250 mL of 10% NaHCO₃ and extracted three times with ethyl acetate. The combined organic phases are evaporated to dryness and dissolved and stirred for 24 h in 25 mL DCM, containing 1M TFA. After evaporation of the solvents, the residue is dissolved in 300 mL of acetone and 3,5 eq of NaOMe are added. The precipitating compound is filtered off, dried and used without further purification. 1g of the tri-sodium salt is dissolved in water, acidified to pH2 and extracted 3 times with chloroform. The pooled organic phases are evaporated to a final volume of 50 mL and to the resulting tri-acid 5 eq. of Pfp-TFA and 20 eq. of DIEA are added and the reaction mixture is stirred for 2h. After the reaction, the mixture is poured into 500 mL of water, extracted 3 times with 200 mL of EtOAc, washed with brine, separated, dried over MgSO4 and evaporated to dryness. The resulting gum is crystallized from hexane / ethyl acetate to give a beige solid. Tris-Pfp ester is dissolved in THF and 5 eq. of the amino-GalNac monomer are added and stirred for 20 min. To the reaction mixture, subsequently 20% Pd/C are added and the reaction is hydrogenated with a balloon for 2h. The reaction mixture is filtered off and evaporated to dryness. The resulting oil is subjected to column chromatography using CHCl₃ / MeOH 9:1 to yield the final trivalent GalNAc construct. The coupling of this trivalent ligand to PLGA follows exactly the same procedure as described in Example 3. The synthesis scheme is shown in Fig. 11.

### Example 3: Coupling of an amino-functionalized Gal-NAc to PLGA as a delivery system.

1g of PLGA (Resomer RG 502 H) MW: 12.000 is dissolved in 5mL of CHCl3 and 8 mg EDCxHCI, predissolved in 1 mL of chloroform and 4.8 mg NHS in 1 mL DMF is added. The activation mixture is stirred for 4h and then a solution of tetraacetyl-GalNAc amine and 80µL of trimethylamine is added under an inert atmosphere. After stirring the mixture at rt for 18h, 58µL of glacial acetic acid is added and the solvents removed under a hard vacuum. The resulting sticky oil is washed 5 times with 50 mL of water and 2 times with 50 mL of methanol. The residue is dissolved in DCM, 0.5 eq. of TFA is added and the reaction stirred for 3h at rt, before all volatiles are removed under reduced pressure. The resulting, deprotected Gal-NAc-PLGA is dissolved in 5 mL of acetone and precipitated in 100 mL of cold water (0°C). The precipitated polymer is filtered off and lyophilized. The Gal-NAc -labelled PLGA is used for a nanoprecipitation procedure or an emulsion procedure for the encapsulation of an appropriate agent. In case of nucleic acid derivatives, a polyplex consisting of PEI or any other basic polymers is formed. Then the encapsulation is performed via double emulsion into the Gal-NAc PLGA. The coupling of amino-terminatd Gal-NAc to PLGA is shown in Fig. 12.

### Example 4: Preparation of nanoparticles

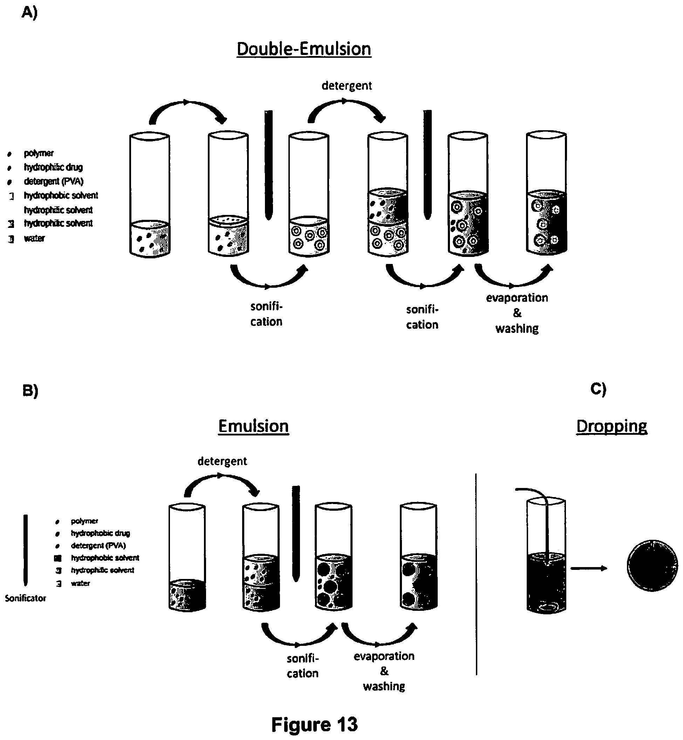

After functionalization of the polymer with the carbohydrate targeting moiety (see Example 5), nanoparticles were produced by nanoprecipitation using polyvinylalcohol (PVA) as surfactant. The polymer and the PKC inhibitor BIM-1 or the PI3K inhibitor AS 605240 are dissolved in DMSO and the solution is slowly dropped into a vigorously stirred aqueous 0.3% PVA solution. The formed nanoparticles contain 4wt% of BIM-1, or 10 wt% of AS 605240 encapsulated in the GalNAc-targeted PLGA. The solution is purified and concentrated by cross-flow filtration. Methods for the preparation of inventive nanoparticles by emulsion, double emulsion and nanoprecipitation is further exemplary illustrated in Fig. 13.

To proof the cell/tissue targeting, neutral-lipid orange (DYOMICS) is encapsulated instead of BIM with an identical procedure. The evaluation and visualization of the hepatocyte targeting is performed according to the intravitalmicroscopic methods of WO2015l035974, the disclosure of which is herewith fully referred to and incorporated.

### Example 5: Characterization of inventive nanoparticles

Nanoparticles of Gal-NAc- PLGA were produced with constant parameters and reproduced according to the protocol as follows:
- Size: Measurement of the size of the various nanostructured delivery systems dissolved in deionized water by dynamic light scatter (for example, Zetasizer (Malvern Instruments GmbH)) or by electron micrographs.
- Shape: Determination of shape by electron micrographs.
- Charge: Measurement of the various nanostructured delivery systems dissolved in deionized water using a Zetasizer (Malvern Instruments GmbH) by determining the electrophoretic signal (zeta potential, surface charge).
- Endotoxins: Endotoxin measurement by LAL chromogenic assay according to D. E. Guilfoyle, et al., Evaluation of a chromogenic procedure for use with the Limulus lysate assay of bacterial endotoxins drug products, J Parenter Sci Technol, 1985, 39(6): pp. 233-6.
- Hemolysis: Measurement of the hemoglobin concentration of erythrocytes which were incubated with the particles in physiological buffer for one hour. The measurable hemoglobin concentration in the supernatant increases when there is damage to the erythrocyte membrane.
- Aggregation: Measurement of the absorption of erythrocytes incubated with the polymers in physiological buffer. Samples with cell aggregates show a lower absorption than homogeneously distributed non-aggregated cells.

**Table 1: Results**

| | **A** | **B** | **C** | **D** |
|---|---|---|---|---|
| **Size [nm]** | 80 | 100 | 93 | 185 |
| **PDI** | 0,13 | 0,10 | 0,21 | 0,18 |
| **Zeta potential** | -12 | -10 | -20 | -17 |

| | | | | |
|---|---|---|---|---|
| **A**: Gal-NAc-targeted nanoparticles from example 4 with 2.5% encapsulated Neutral-lipid orange: **B**: Untargeted nanoparticles (PLGA/PVA) with 2.5% encapsulated Neutral-lipid orange **C**: Gal-NAc-targeted nanoparticles from example 4 with 4% BIM (PKC inhibitor) **D**: Gal-NAc-targeted nanoparticles from example 4 10% AS605230 (PI3 kinase inhibitor) | | | | |

### Example 6: Static macrophage assay and dynamic chip based microfluidic model for hepatocyte targeting and interaction with macrophages

The Macrophage assay was used to investigate if any unwanted uptake and/or effect of nanoparticles by macrophages occur. Interactions between NPs and macrophages can seriously reduce the efficacy of NPs. In addition, interaction can result in activation of macrophages, thereby harming the surrounded tissue, after all the host. Therefore, the interaction between NPs and macrophages should be proven first. Particle size, shape and coating and surface charge are critical determinants. Two assays were performed under static conditions:

### A. Human peripheral blood mononuclear cell (PBMC) culture and macrophage differentiation

PBMCs were freshly isolated immediately after collecting donor blood from healthy volunteers. The donors were informed about the aim of the study and gave written informed consent. Blood sample volume was diluted in a ratio 1:1 with PBS without calcium and magnesium (Biochrom AG, Germany) containing 0.1% bovine serum albumin (BSA, Carl Roth, Germany) and 2 mM EDTA (Sigma-Aldrich, Germany; isolation buffer) and carefully laid on top of Biocoll separating solution (Biochrom AG, Germany). PBMCs were obtained from density gradient centrifugation. The cells were washed subsequently in isolation buffer for several times and were finally strained by a 40 µm molecular mesh (BD Bioscience, Germany). For monocyte enrichment 10⁷ PBMCs per well (9,6 cm²) were plated on a six well plate (or in smaller wells with comparable cell density) in 2 mL X-VIVO 15 (Lonza, Germany) supplemented with 10% autologous serum, 10 ng/ mL GM-CSF (PeproTech, Germany), 100 units/ mL penicillin, and 100 µg/ mL streptomycin (Life Technologies, Germany). The cells were washed with plain X-VIVO 15 medium after 3 h of incubation and fresh medium with supplements (stated above) was added. Including the preparation time for nanoparticle experiments, macrophage (Mφ) differentiation was performed for five days.

### A1. Murine macrophage cell line RAW264.7 culture and differentiation

RAW 264.7 macrophages (CLS, Eppelheim, Germany) were cultivated in 75 cm² cell culture flasks in RPMI 1640 medium supplemented with 2 mM L-glutamine, 10% fetal bovine serum and 100 units/ mL penicillin, and 100 µg/mL streptomycin at 37°C in humidified 5% CO₂/ 95% air atmosphere. Media exchange was performed after 2-4 days (depending on cell confluency). For experiments macrophages were detached by Accutase treatment and were seeded, cultured for 24 hours and then incubated with particles (i.e. NPs with loaded neutral lipid orange in phenol-red free medium for individual time periods. After incubation macrophages were harvested and/or lysed followed by individual analysis (i.e. by a microplate reader with fluorescence detection system). Protein contents were analyzed using BCA Assay (Thermo Fisher Scientific, USA)

To achieve more meaningful data compared to static mono-cell culture, several scalable co-culture-models were used. They resemble the *in vivo* situation better than static mono-cell cultures:

### A2. Co-Culture of endothelial cells and macrophages

According to Rinkenauer AC et al., Comparison of the uptake of methacrylate-based nanoparticles in static and dynamic in vitro systems as well as in vivo, J Control Release. 2015; 216:158-68, Nanoparticle (NP) were tested in co-culture model of endothelial cells and macrophages under physiologic shear stress conditions. Briefly, monocytes were harvested 24 h after isolation by treatment with 4 mg mL⁻¹ lidocaine (Sigma-Aldrich, Germany) and 5 mM EDTA. Confluent HUVECs were detached using trypsin. Monocytes were stained with 1 µM CellTracker green CMFDA (Life Technologies, Karlsruhe, Germany) for 45 min in serum-free X-VIVO 15. Subsequently, monocytes and HUVECs were pooled 1:3 in Endothelial Growth Medium MV supplemented with 10% autologous serum, 10 ng mL⁻¹ GM-CSF and 100 UmL⁻¹ penicillin and 100 µgmL⁻¹ streptomycin and seeded at a density of 1.3 × 10⁵ HUVECs cm-2 and 0.43 × 10⁵ monocytes cm² into rhombic chamber chips. Medium was changed on a daily basis. Mφ differentiation was performed in presence of GM-CSF for 72 h under static culture conditions. HUVEC were perfused using peristaltic pumps (Ismatec REGLO digital MS-CA-4/12-100, Germany). Shear stress within rhombic chamber chips was calculated as previously described (Microfluidically supported biochip design for culture of endothelial cell layers with improved perfusion conditions. Raasch et al,; Biofabrication, 2015, 7(1):015013). Shear stress of 0.7, 3.0, 6.0 and 10.0 dyn cm⁻² was applied for 24 h following 60 min nanoparticle uptake at a concentration of 200 µg mL⁻¹. Negative charged nanoparticles containing nile red were solved in Endothelial Cell Growth Medium MV without additives

### B. Dynamic42 Sinusoid - Chip based microfluidic model

Cell specificity and targeting is determined in a chip based microfluidically supported multi-cell culture system consisting of macrophages, hepatocytes, stellate cell and, endothelial cells. According to Rennert K. et al, A microfluidically perfused three dimensional human liver model, Biomaterials 2015; 71:119-131, the cell culture and assembling of the Dynamic42 Sinusoid - model was performed:

### HepaRG and endothelial cell preparation for Dynamic42 Sinusoid model

HepaRG cells were seeded at a density of 2.7 x 10⁴ cells/cm² and cultured in William's Medium E (Biochrom, Berlin, Germany) containing 10% (v/v) FCS (Life Technologies, Darmstadt, Germany), 5 µg/ml insulin (Sigma Aldrich, Steinheim, Germany), 2 mM glutamine (GIBCO, Darmstadt, Germany), 50 µM hydrocortisone-hemisuccinate (Sigma-Aldrich) and 100 U/ml Penicillin/100 mg/ml Streptomycin mixture (Pen/Strep) (GIBCO). The cells were cultured in a humidified cell incubator at 5% CO₂ and 37° C for 14 days before differentiation. Medium was renewed every 3 - 4 days. Cell differentiation was induced and cells were used up to 4 weeks.

Endothelial cells: Human umbilical cord vein endothelial cells (HUVECs) were isolated from human umbilical cord veins. Donors were informed about the aim of the study and gave written consent. HUVEC cells were seeded at a density of 2.5 10⁴ cells/cm² and cultured in Endothelial Cell Medium (ECM) (Promocell, Heidelberg, Germany) up to passage 4.

### LX-2 stellate cell and macrophage preparation for Dynamic42 Sinusoid model

LX-2 stellate cells (kindly provided by Scott L. Friedman, Division of Liver Diseases, Mount Sinai School of Medicine, New York City, NY, USA) were seeded at a density of 2.0 x 10⁴ cells/cm² and cultured in Dulbecco's Minimum Essential Medium (DMEM) (Biochrom) supplemented with 10% (v/v) FCS, 1 mM sodium pyruvate (GIBCO) and Pen/Strep. Peripheral Blood Mononuclear Cells (PBMCs) were isolated by Ficoll density gradient centrifugation and seeded at a density of 1.0 x 10⁶ cells/cm² in X-VIVO 15 medium (Lonza, Cologne, Germany) supplemented with 10% (v/v) autologous human serum, 10 ng/ml human granulocyte macrophage colony-stimulating factor (GM-CSF) (PeproTech, Hamburg, Germany) and Pen/Strep. After 3 h incubation in a humidified cell incubator at 5% CO₂ and 37°C the cells were washed twice with X-VIVO 15 medium. Adherent monocytes were cultivated for 24 h in X-VIVO 15 medium and seeded into the liver sinusoid.

### Assembly of the Dynamic42 Sinusoid

Liver sinusoid models were assembled by staggered seeding of vascular and hepatic cell layers. In each sterilized biochip 2.7 x 10⁵ HUVEC's/cm² (in total 3.0 10⁵ cells) and 0.9 x 10⁵ /cm² Monocytes (in total 1 x 10⁵ cells) were mixed and seeded on top of the membrane in the upper chamber. HUVEC/monocytes were co-cultured for at least 3 days with a daily medium exchange in endothelial cell culture medium (ECM) supplemented with 10 ng/ml epidermal growth factor, 90 mg/ml heparin, 2.8 mM hydrocortisone, endothelial cell growth supplement, 10 ng/ml GM-CSF, 10 ng/ml M-CSF to induce macrophage differentiation, 100 U/ml penicillin/100 mg/ml streptomycin and 10% (v/v) autologous human serum (Life Technologies, Karlsruhe, Germany). Subsequently, 2.7 x 10⁵/cm² differentiated HepaRG (in total 3 x 10⁵ cells) and 0.9 x 10⁴/cm² LX-2 (in total 1 x 10⁴ cells) were seeded on the membrane at the opposite side of HUVEC cells and cultured for 24 h in DMSO-free William's Medium E (Biochrom, Berlin, Germany) hepatocyte growth medium containing 50 µM hydrocortisone, 10% (v/v) FBS containing, 5 µg/ml insulin, 2 mM glutamine and 100 U/ml penicillin/100mg/ml streptomycin prior to experimental use.

**Table 2: Dimensions of the sinusoid chip**

| | *length* / *width* / *height (mm)* |
|---|---|
| chip body | 75.5 / 22.5 / 1.5 |
| upper channel | 15.0 / 2 / 0.45 |
| lower channel | 16.8 / 2 / 0.40 |
| membrane (8 µm pore diameter) | 13 / 8.5 / 0.02 |

| | *distance (mm)* |
|---|---|
| membrane to upper sealing foil | 0.7 |
| membrane to lower sealing foil | 0.8 |

**Table 3: Flow rates within the sinusoid chip**

| | *flow rate (µl* / *min)* | *shear stress ((dyn* * *s)* / *cm²)* |
|---|---|---|
| upper channel | 50 | 0.7 |
| lower channel (as indicated in corresponding experiments) | 1 | 0.01 |
| | 3 | 0.03 |
| | 10 | 0.12 |

Liver sinusoid models were equilibrated after 7 days in static culture by perfusion with a flow rate 50 µl/min for up to 72 hours. Subsequently, drug constructs and controls (at least triplicates) were incubated for individual time periods in the liver sinusoid model under variable dynamic conditions. Afterwards liver sinusoids were fixed by paraformaldehyde or methanol or both and analyzed by immunofluorescence staining. The different cell layers were examined with a fluorescence microscope to analyze the enrichment of the constructs in or on different cell types. In addition, it is possible to lyse the vascular and hepatic cell layer separately and to measure the cell-specific uptaken nanoparticle by a microplate reader with fluorescence detection system.

### Example 7: Determination of the cytotoxicity

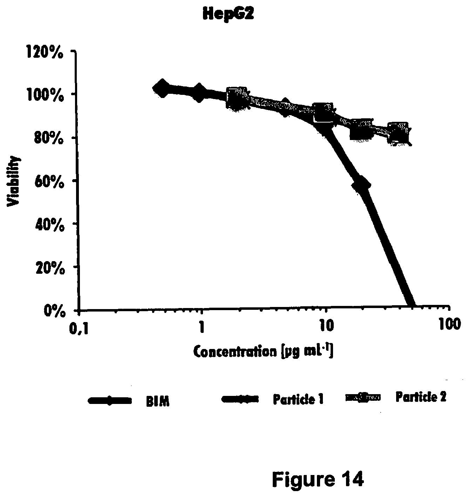

Cytotoxicity studies were performed with the mouse fibroblast cell line HepG2 (human liver cancer cell line), as recommended by ISO10993-5. Cells were seeded at 104 cells per well in a 96-well plate in Dulbecco's modified eagle's medium (DMEM, Lonza, Basel) supplemented with 10% fetal calf serum (FCS), 100 U/mL penicillin and 100 mg/mL streptomycin and incubated for 24 h at 37 C° in a humidified 5% (v/v) CO2 atmosphere. The testing substances (polymers) at indicated concentrations (from 0.5 µg/mL to 50 µg/mL) were added to the cells and the plates were incubated for further 24 h. Control cells were incubated with fresh culture medium. Subsequently, the medium was replaced by a mixture of fresh culture medium and Alamar-Blue solution (Life technologies, Darmstadt, Germany), prepared according to the manufacturer's instructions. After a further incubation of 4 h at 37°C, the fluorescence was measured at Ex 570/Em 610 nm, with untreated cells on the same well plate serving as negative controls. The negative control was standardized as 0% of metabolism inhibition and referred as 100% viability. Cell viability below 70% was considered indicative of cytotoxicity. Data are expressed as mean ± S.D. of three determinations. Fig. 14 shows that the encapsulated drug is less toxic in higher concentrations compared to the free drug (BIM-1).

### Example 8: Survival-rate in cholestasis model under septic conditions "Peritoneal Contamination and Infection (PCI)"

A systemic infection / sepsis with organ failure is induced by using the PCI model. For this purpose, a human faeces suspension (2.5 mu.l / g body weight) is injected intraperitoneally (without anesthesia) with weight adaptation, thus triggering peritonitis with subsequent systemic infection. In order to avoid the burden on the animals and a dying 6 hours after infection twice daily also weight-adapted volume (Ringer's lactate) in combination with a broad-spectrum antibiotic (Meropenem) administered subcutaneously (neck fold). The administered dose of antibiotic (or combination) used amounts to 2.5 µg / g body weight (meropenem), the volume to 10 µl / g body weight. The animals are examined in this model for the first 5 days (acute phase of infection) closely every 3 hours for signs of infection in order to timely, according to defined endpoints, to be able to exclude the animals in advance from the experiment. After 5 days, the animals show no health impairment, so that no increased burden on the animals for the remainder of the observation period is assumed. The examination of the animals therefore takes place from day 6 twice a day. Should animals still show signs of disease after 5 days, close monitoring will continue.

For dose determination, three drug concentrations per formulation are tested in small groups and changes in survival are documented. The PI3K inhibitor AS605240 and the PKC inhibitor Gö6850 (aka BIM1) are tested in three concentrations 0.17, 0.5 and 1 mg/kg body weight (BW) in two formulations with a small group size. By formulating the active ingredients in nanoparticles / liposomes, the amount of active substance in immune cells should be reduced by a targeted accumulation of these in hepatocytes. In addition, possible immunosuppressive side effects of the inhibitors should be minimized or suppressed. The dose optimization of the respective two formulations containing active ingredient is carried out independently. In the best case, the optimal dose for the nanoparticle formulations is less than that of the free drug. However, this does not allow a final decision as to which formulation is "better".

Six hours after infection (PCI model), the therapy is carried out with different drug or control formulations (once daily, i.p. or i.v.) and also the combined volume and antibiotic therapy (twice daily, s.c.). The therapy with the drug is scheduled for 5 days. The volume / antibiotic therapy takes place over 7 days (2 days longer than the drug therapy). The observation in the first 5 days is performed in a 3-hour interval for 24 hours a day. This is followed by observation of the animals until day 14 (twice a day). (Results not shown).

## Claims

1. Inhibitors of the PKC signaling pathway for use in the treatment of septic cholestasis, wherein the inhibitors are targeted into the liver by a selective nanostructured delivery system, wherein the selective nanostructured delivery system comprises at least one carbohydrate targeting moiety and at least one polymer and/or at least one lipid and/or at least one virus-like particle.

2. Inhibitors of the PKC signaling pathway for use according to claim 1, wherein the carbohydrate targeting moiety is selected from the group consisting of *N*-acetyl-galactosamine (GalNAc), galactose, lactose, mannose, glucosamine, asialofetuin, pullulan, arabinogalactan, glycorrhizin, glycorrhetinic acid and derivatives thereof.

3. Inhibitors of the PKC signaling pathway for use according to claim 1 or 2, wherein the carbohydrate targeting moiety binds to a recognizing unit located on the liver.

4. Inhibitors of the PKC signaling pathway for use according to claim 3, wherein the recognizing unit is a receptor, preferably a lectin, more preferably the asialoglycoprotein receptor (ASGPR) or aka Ashwell-Morell receptor.

5. Inhibitors of the PKC signaling pathway for use according to one of the preceding claims, wherein the inhibitors are selected from the group consisting of PKC inhibitors, PI3 kinase inhibitors, MAPK inhibitors, PLC inhibitors, DAG level reducing agents, siRNA, shRNA, miRNA, modified oligo analouges, antisense constructs, and RNAse H.

6. Inhibitors of the PKC signaling pathway for use according to claim 5, wherein the inhibitors are PKC inhibitors selected from the group consisting of bisindolylmaleimides, staurosporine, midostaurin, UCN-01, sotrastaurin, enzastaurin, ruboxistaurine, tivantinib, enzastaurin, Gö 6983, K252a, ANA-12, lestaurtinib, stauprimide, CEP-701, Arcyriaflavin a, and Bisindolylmaleimids I-XII aka BIM I-XII.

7. Inhibitors of the PKC signaling pathway for use according to claim 5, wherein the inhibitors are PI3 kinase inhibitors selected from the group consisting of copanlisib, idelalisib, wortmannin derivatives, bryostain derivatives, taselisib, omipalisib, AS605240, GSK1059615, buparlisib, alpelisib, pictilisib, serabilisib, dactolisib, dihydrosphingosine, calphostin C and melittin.

8. Inhibitors of the PKC signaling pathway for use according to any of the preceding claims, wherein the inhibitor directly or indirectly inhibit or reduce the activity of PKC or PKC subtypes.

9. Inhibitors of the PKC signaling pathway for use according to any of the preceding claims, wherein the at least one polymer is selected from the group consisting of polyesters, polyacrylates, polystyrene derivatives, polyamides, polyurethanes, polyacrylonitriles, polytetrafluoroethylenes, silicones, silica particles, cerium oxid aluminium oxide or apatite particles, polyethylene glycols, polyethylene oxides and polyoxazolines and their copolymers, preferably in a variety of compositions such as random, gradient, alternating, block, graft or star copolymers.

10. Inhibitors of the PKC signaling pathway for use according to claim 9, wherein the at least one polymer is an organic, inorganic, hydrophobic, hydrophilic, amphiphilic, anionic and/or cationic polymer.

11. Inhibitors of the PKC signaling pathway for use according to claim 9 or 10, wherein the at least one polymer is selected from the group consisting of PLGA, PLA, PCL, PGA, PDMAEMA, PMMA, PMAA, PEI, PEtOx, PEG, HPMA, APMA, PVP, hydrolyzed PVP and polysaccharides.

12. Inhibitors of the PKC signaling pathway for use according to any of the preceding claims, wherein the at least one lipid is selected from the group consisting of saturated and unsaturated fatty acids, cholesterol derivatives, phospholipids, sphingolipids, lipoproteins and glycolipids.

13. Inhibitors of the PKC signaling pathway for use according to any of the preceding claims, wherein the at least one virus-like particle is derived from a virus selected from the group consisting of Bacteriophage MS2, Bacteriophage Qβ, Enterobacteria phage P22, Cowpea mosaic virus (CPMV) Cowpea Chlorotic Mottle Virus (CCMV), hepatitis B virus carries (HBVc) and Adeno associated virus (AAV).
